# EUROPEAN PATENT SPECIFICATION

(11) **EP 1 002 054 B1**
(45) Date of publication and mention of the grant of the patent: **19.10.2005**
(21) Application number: 99915384.4
(22) Date of filing: 17.03.1999
(51) Int. Cl.: C12N 5/06, C12N 7/02

(54) **PROCESS FOR THE PRODUCTION OF VIRUS IN CELL CULTURES**
VERFAHREN ZUR HERSTELLUNG VON VIREN AUS ZELLKULTUREN
PROCEDE DE PRODUCTION DE VIRUS DANS DES CULTURES CELLULAIRES

(30) Priority: 18.03.1998 BR 9804283
(43) Date of publication of application: 24.05.2000
(73) Proprietor: FUNDACAO OSWALDO CRUZ (FIOCRUZ), 21045-900 Rio de Janeiro (BR)
(72) Inventor: DA SILVA FREIRE, Marcos, Rua Cândido Port., Pendotiba, CEP-24320-000 Niteroi, RJ (BR); YAMAMURA, Anna, Maya, Yoshida, Pendotiba, CEP-24320-000, Niteroi,RJ (BR); MANN, George, Fobes, CEP-21045-900 R.de Janeiro, RJ (BR); GALLER, Ricardo, Rua Cândido Port., Pendotiba, CEP-24320-000 Niteroi, RJ (BR)
(74) Representative: Woods, Geoffrey Corlett
(86) International application number: PCT/BR1999/000014
(87) International publication number: WO 1999/047645

(56) References cited:
- EP-B1- 0 551 449
- WO-A1-91/09937
- US-A- 5 650 318

## Description

The present invention relates to the production of vaccine viruses in cell cultures at high virus yields, particularly for the production of Flaviviruses in fibroblast cell cultures, and more particularly for production of any substrain of the 17D yellow fever virus in culture of chick embryo fibroblasts. The virus present in a vaccine preparation can be obtained by inactivation, attenuation and genetic modification through recombinant DNA technology.

### BACKGROUND OF THE INVENTION

Conventional vaccines are usually prepared by producing large amounts of virus in cell tissue culture or in laboratory animals and then rendering the virus harmless without destroying its immunological properties Traditionally, this is accomplished either by inactivating the infectivity of the virus or by selecting an avirulent or attenuated virus for use in live vaccines. Attenuation is often established by adapting the virus to unusual growth conditions (i.e., different animal hosts or cell cultures) and by frequent passages in cell culture of large viral inocula to select mutants which have lost virulence and thus produce minor or no clinical symptoms.

On serial passage in cell cultures, attenuated vaccine viruses can potentially revert to the virulent phenotype by mutation or selection. For a subpopulation in the original virus preparation, it is common practice therefore to use a seed lot system in which an original virus preparation, shown to be attenuated for man, is passaged once to give multiple aliquots of primary (master) seed. One or more aliquots of virus from this primary seed is passaged once further to give a secondary (working) seed preparation. One or more aliquots of this secondary seed preparation are used in the production of one batch of vaccine. In this way, serial passage can be limited and the probability of selection or mutation minimised. An example of this method to obtain attenuated vaccine viruses is the production of Yellow Fever (YF) vaccine strains.

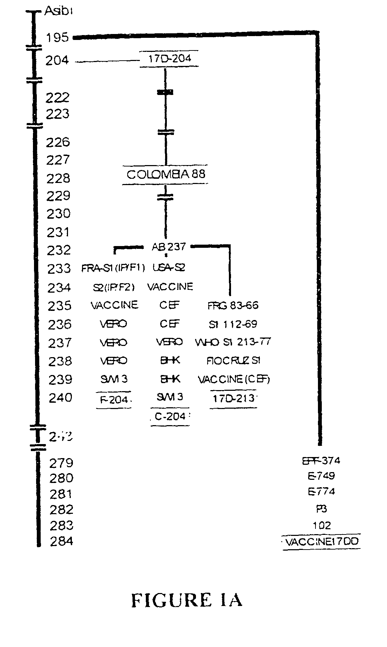

In Figure 1A the passage history of the original YF Asibi strain and derivation of YF 17D vaccine strains and its substrains is depicted. The YF virus Asibi strain was subcultured in embryonic mouse tissue and minced whole chicken embryo with or without nervous tissue. These passages yielded the parent 17D strain at passage level 180. 17DD at passage 195 and 17D-204 at passage 204. 17DD strain was further subcultured until passage 241 and underwent 43 additional passages in embryonated chicken eggs at passage 284 which corresponds to the vaccine batches produced nowadays in use for human vaccination. The 17D-204 was further subcultured to produce Colombia 88 strain which, upon passage in embryonated chicken eggs, gave rise to different vaccine seed lots currently in use in France (I. Pasteur, at passage 235), in the United States (Connaught, at passage 234). The 17D-213 strain was derived from 17D-204 when the primary seed lot (S1 112-69) from the Federal Republic of Germany (FRG 83-66) was used by the World Health Organization (WHO) to produce an avian leukosis virus-free 17D seed (S1 213/77) at passage 237. New 17D substrains have been developed through the use of recombinant DNA technology which required a better knowledge on flavivirus genome structure and expression. The Yellow Fever virus is the prototype virus for the Flavivirus genus within the *Flaviviridae* family of positive-stranded RNA viruses, The flaviviruses are spherical viruses with 40-60 nm in diameter with an icosahedral capsid which contains a single positive-stranded RNA molecule and viral replication takes place entirely in the cell cytoplasm. The YF virus RNA genome consists of 10,862 nucleotides in length with short 5' (118 nucleotides) and 3' (511 nucleotides) untranslated regions, a 5 cap structure and nonpolyadenylated 3' end. This single RNA is also the viral message and its translation in the infected cell results in the synthesis of a polyprotein precursor which undergoes posttranslational, proteolytic processing to generate 10 virus-specific polypeptides. In order to manipulate RNA genomes at any particular site, complementary DNA's corresponding to the complete genome must be available. The pioneer study of Rocaniello and Baltimore (Rocaniello. V.R. and Baltimore, D. (1981) "Cloned poliovirus complementary DNA is infectious in mammalian cells". Science. 214: 916-919) first showed the feasibility to generate virus from cloned cDNA. With the development of *in vitro* transcription systems (see Melton. D.A., Krieg, P.A., Rabagliati. M.R.: Maniatis. T., Zinn. K. and Green, M.R. (1984). "Efficient in vitro synthesis of biologically active RNA and RNA hybridization probes from plasmids containing a bacteriophage SP6 promoter" Nucl. Acids. Res. 12: 7035-7056) it became possible to synthesize *in vitro* full-length viral RNA with a much higher etliciency as compared to cDNA transcription in the cell. The development of more efficient transfection methodologies such as cationic liposomes and electroporation helped improve the efficiency of transfection of cultured cells with RNA. The basic methodology for what is known today as infectious clone technology was set. For a number of positive stranted viruses, infectious cDNA has been obtained and can be used to understand the molecular basis of several biological phenomena including attenuation. A full-length cDNA was first developed for YF virus and demonstrated to give rise to a virus after transfection of cultured vertebrate cells with *in vitro* synthesized RNA in 1989 (Rice *et al,* 1989).

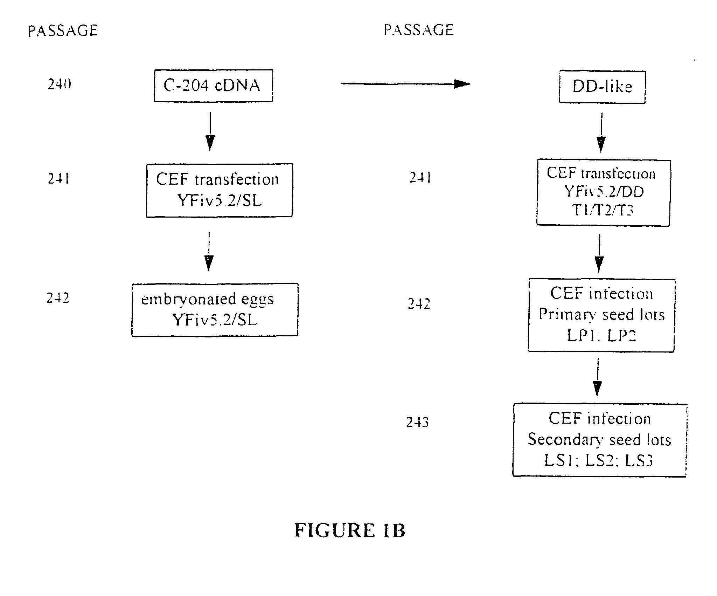

The derivation of YF infectious clone progeny viruses is depicted in Figure 1B. The viral RNA of 17D substrains boxed in Figure 1, named F-204, C-204, 17D-213 and vaccine 17DD have been the subject of complete nucleotide sequence determination by different groups and published elsewhere. Each of the 17D-204 substrains, F-204 and C-204, was plaque purified in different cell lines, and the obtained plaque-purified virus was amplified in SW13 cells and used for cDNA cloning and sequence analyses. For 17D-213 and 17DD viruses. RNA was extracted directly from virus which had been purified from the vaccine preparation, and these populations are therefore more likely to represent the sequence which should confer an attenuated phenotype to the vaccine virus. The only YF infectious cDNA derived to date was originated from the C-204 cDNA library (Rice, C.M.; Grakoui. A.; Galler. R. and Chambers. T. (1989). "Transcription of infectious yellow fever RNA from full-length cDNA templates produced by *in vitro* ligation". The New Biologist. **1**: 285-296) but the charaterization of virus recovered from it after transfection in CEF cells (YFiv5.2/SL at passage 241) and one passage in embryonated eggs using current manufacturing methodology (YFiv5.2/VL at passage 242) revealed a virus with an unacceptable degree of neurovirulence for nonhuman primates and therefore not suitable to be tested for human vaccination (Marchevsky R.S., Mariano, J., Ferreira, V.S., Almeida, E., Cerqueira. M.J., Carvalho, R., Pissurno, J.W., Travassos da Rosa, A.P.A., Simões.,M.C., Santos, C.N.D., Ferreira, I.I., Muylaert, I.R., Mann, G.F., Rice, C.M. and Galler, R., "Phenotypic analysis of yellow fever virus derived from complementary". DNA Am. J Trop. Med. Hyg., 52(1), pp. 75-80. 1995). Comparison of the nucleotide sequences for the 17D-213 and 17DD substrains depicted in Fig. 1A led to the genetic modification of the cDNA of C-204 virus rendering it more DD-like. This new cDNA was used to direct the *in* *vitro* synthesis of RNA which upon transfection of CEF cells gave rise to a new seed virus named YFiv5 2/DD at passage 241. This virus was then used to prepare the primary and secondary seed lots, both in CEF, at passages 242 and 243, respectively, as described in the Patent Application BR PI 9701774, filed in the name of the same applicant of the present invention. In short, given the well known vaccine properties of YF 17D virus with regard to safety and efficacy, the capability to manipulate its genome paved the way to its development as a vector to direct the expression of antigens from other pathogens.

Regardless the vaccine virus is obtained by inactivation, attenuation or the use of recombinant DNA techniques, the virus must be compatible with the host cell, i.e., capable of autonomous replication in the host cell. Moreover, in the large-scale production, the host cell must be a cell insofar as the virus can grow there with high yield. Cells for the production of live virus vaccines for parenteral use in man are limited to those which are free of adventitious agents, non-tumorigenic and karyologically normal. Such cells include primary or serially passaged cultures derived from a variety of mammalian or avian tissues such as human diploid fibroblasts (e.g., MLRC-5), monkey kidney (MK), avian embryo fibroblasts (e.g., chick embryo fibroblasts) and many others. These cells are infected with the virus ("seed virus") which then replicates in the cell and is usually released into the culture medium. The conditions and materials used in the whole process must be carefully monitored to avoid problems mainly related to: genetic variability, loss of immunogenicity, virus inactivation, neurovirulence, low yield in relation to virus input and contamination by extraneous agents.

In the US 5,391,491 and US 5,550,051, it is proposed a method of producing virusivirus antigen, in particular the flavivirus tick-borne encephalitis virus, by infecting a biomass comprised of avian embryo cells aggregates having diameters of between 100 µm and 1.000 µm with the said virus in a culture medium having an oxygen concentration of at least 0.01 mmol/l, the cell aggregates being present in a concentration of at least 10 mg of cell aggregates per ml. The applicant mentioned that the control of the aggregates size and the oxygen concentration aims both to reduce the contamination of cell proteins in the medium caused by the gradual cell lysis and to increase the reproducibility of the virus/virus antigen which is low when individual cells or small aggregates are present in suspension. It is also described that measurements demonstrated that TBE-virus/virus antigen production will be strongly reduced on a larger scale in a culture having a cell density of 2 x 10⁷ cells per milliliter that communicates with the oxygen atmosphere exclusively by its surface.

Several attempts have also been made to preserve the immunogenicity of the vaccine virus. US 5,021,347 describes a recombinant vaccinia virus expressing the E-protein of Japanese encephalitis virus aiming to avoid the decay of the immunogenic properties of the vaccinal virus. The animal culture cell used in the infection with the virus is any cell insofar as vaccinia virus can grow there. Specific examples of culture cell cited in the patent include TK-143 (derived from human osteosarcoma). FL (derived from human amnion). Hela (derived from carcinoma of human uterine cervix). KB (derived from carcinoma of human rhinopharynx). CV-1 (derived from monkey kidney), BSC-1 (derived from monkey kidney). RK13 (derived from rabbit kidney), L929 (derived from mouse connective tissue). CE (chicken embryo) cell. CEF (chicken embryo fibroblast) cells.

In addition, in Marchevsky *et al* (Marchevsky *et al.* 1995), primary culture of certified SPF (Specific Pathogen Free) chicken embryo fibroblasts (CEF) for human vaccine production are mentioned as appropriate in the preparation of the master seed lot of a recombinant YF vaccine virus.

Despite the advantages of using certified SPF-CEF cells to produce vaccine viruses, such as YF, measles, mumps, rubella, it is frequently found that the process is relatively uneconomic due to low yields. This is particularly true for the YF vaccine virus produced in CEF at the cell densities normally used (1 x 10⁶ cells/cm²) where yield is directly related to virus input. Thus, the amount and cost of the seed virus necessary under these conditions is too high due to the need for costly neurovirolence tests on each seed lot.

The conditions for optimal virus production must be carefully controlled in terms of incubation temperature and time, cell seeding density and virus inoculum. In addition, a number of other factors determine virus yield. Thus, some viruses readily generate defective interfering particles which are avoided by using low virus inputs. Some viruses are good inducers of interferon and may be sensititive to its action. The stability of virus in the culture medium which is being used is also of vital importance since yield is a balance between production and loss of infectivity.

The role of interferon in the virus/cells system has been studied since interferon was discovered by Isaacs and Lindemann (Isaacs. A. and Lindemann. J. Proc. Rov. Soc. Ser. B., 147: 258 1957). Interferons are materials having antiviral properties and are produced by certain types of cells which have been stimulated by exposure to a virus, certain nucleic acids, or antigen/mitogen complexes.

Mammalian and avian interferons fall into three main categories, alpha, beta and gamma. Alpha interferon is produced by leucocytes, beta by fibroblasts and gamma by T lymphocytes and natural killer cells stimulated by alloantigens, tumors, and mitogens. All three are secreted by the respective cells after the cells are stimulated by a virus or other inducer. The interferons are frequently referred to the cell of origin and, therefore, gamma interferon is also known as "immune" interferon. Interferon activity is traditionally determined based on its ability to induce resistance in cells which where challenged with an interferon sensitive virus. In the production of vaccine viruses in cell cultures, the interferon system is responsible for low virus yields of some vaccine viruses since they may be good inducers of interferon and/or sensitive to its action. The 17D yellow fever vaccine virus is both a good inducer of interferon and extremely sensitive to its action.

Indeed, the interferon activity is the ability of cells to resist to a virus challenge. Thus interferons are potentially potent drugs which show promise as clinical antiviral agents.

If the antiviral activity of the interferons is an advantage in relation to the treatment of humans against diseases caused by virus, it is a disadvantage when dealing with the *in vitro* production of virus because the interferons inhibit, specifically, the replication of virus in cells. This is probably the main cause of the low yields in the production of many vaccine viruses.

Consistently, the FR 2 689 519 patent application proposes a process of *in vitro* culturing cells infected with a virus associated with sclerosis in which a primary cell culture is infected with the virus in a culture medium containing polyclonal antibodies anti-interferon-beta to inhibit the antiviral activity of the interferon-beta. The disadvantage of the process is the addition of contaminant antibodies which can be difficult to separate from the desired product, thus limiting its usefulness in vaccine virus production.

Accordingly, there is a great need for an improved process to produce interferon inducing/sensitive vaccine viruses without the disadvantages mentioned above.

### Summary of the Invention

The object of the invention is a process for the production of Flavivirus in cell culture with high yield and low levels of contamination by cell proteins, the Flavivirus being appropriate to be used in the preparation of seed lots and vaccines. As used herein, the term "virus" means inactivated virus, attenuated virus and recombinant virus, including chimeric virus. Particularly, the chimeric virus may have a genome comprising nucleic acid sequences encoding, at least, one structural protein from one flavivirus and nucleic acid sequences encoding the remainder of the genome of another flavivirus to make it functional. More particularly, the chimeric virus may have a genome comprising nucleic acid sequences encoding, at least, one structural protein from one flavivirus and nucleic acid sequences encoding the remainder of the genome of YF 17D virus to make it functional.

In one embodiment, the present invention relates to a process for the production of a Flavivirus in a cell culture, the process comprising: (a) preparing a culture of cells which are permissive to the virus and acceptable as a substrate for vaccine production; (b) suspending the cells in a suitable medium followed by cell seeding at a density lower than 2x10⁵ cells/cm²; (c) incubating the cell culture at 30 to 40°C for a period of time between 12 and 72 hours; (d) removing the medium from the cell culture of step (c) and inoculating the cells with seed virus; (e) incubating the cell culture of step (d) at 20 to 40°C for a period of time between 12 and 144 hours; (f) removing the medium, washing the cells one or more times and replacing the medium; (g) incubating the cell culture of step (f) at 30 to 40°C for a period of time between 12 and 144 hours; (h) at least partially harvesting the culture supernatant containing virus; and (i) storing the virus at a temperature of -45°c or lower. The cell culture may alternatively be incubated at 25 to 40°C at incubation steps (e) and (g).

In another embodiment, the present invention relates to a process for the production of a recombinant yellow fever (YF) virus in a cell culture, the process comprising: (a) preparing a culture of cells which are permissive to the virus and acceptable as a substrate for vaccine production; (b) cell seeding at a density lower than 2x10⁵ cells/cm²; (c) transfecting the cells with nucleic acids suitable for the production of a recombinant YF 17D virus in a cell culture, by treatment with synthetic lipids or electroporation; (d) incubating the cell culture at 30 to 40°C for a period of time between 12 and 72 hours; (e) removing the medium from the cell culture of step (d) and inoculating the cells with seed virus; (f) incubating the cell culture of step (e) at 20 to 40°C for a period of time between 12 and 144 hours; (g) removing the medium, washing the cells one or more times and replacing the medium; (h) incubating the cell culture of step (g) at 30 to 40°C for a period of time between 12 and 144 hours; (i) at least partially harvesting the culture supernatant containing virus; and (j) storing the virus at a temperature of -45°C or lower. The cell culture may alternatively be incubated at 25 to 40°C at incubation steps (f) and (h).

The present invention represents a breakthrough in the production of seed lots and vaccine virus and is characterized by a fine control of the conditions and starting materials, such as the density and type of cells to be infected with virus, virus input and use of stabilizers.

### BRIEF DESCRIPTION OF THE DRAWINGS

- FIGURE 1: illustrates the passage history of the original YF Asibi strain and derivation of YF 17D vaccine strain and its substrains (A), and the YF infectious clone progeny viruses (B)
- FIGURE 2: illustrates the YF 17D virus strain yield related to the incubation time (A), the level of induced interferon in relation to the incubation time (B) and the degree of resistance to Sindbis challenge with the incubation time (C).
- FIGURE 3: shows the interferon mediated inhibition of Sindbis virus in cultures of chick embryo fibroblast cells infected with the YF 17D virus strain. The cultures were infected with serial dilutions of YF virus and incubated at 37°C. 5 hours later (A). 24 hours (B) and 72 hours (C) the cultures were submitted to Sindbis virus action.
- FIGURE 4: shows the influence of the pretreatment of the cells with actinomycin D: referring to the induced interferon with the incubation time (A) and referring to the YF 17D virus strain yield with the incubation time.
- FIGURE 5: diagrammatically shows the prototype structure of the Flavivirus genome and the structure of chimeric viruses obtained by using the methology of the present invention.

### DETAILED DESCRIPTION OF THE INVENTION

Several factors can influence the yield of virus. The high degree of purity required for potentially administrable vaccines limits the alternative methods which can be applied to virus yield improvement.

In the International Symposium on Yellow Fever and Dengue - Fifty years from the introduction of the 17D strain in Brazil, Rio de Janeiro. Brazil. May 15-19, 1988, it was discussed the necessity of improving the process of production of YF vaccinal virus. Lopes. O.S., (Lopes, O.S. "Development of a Yellow Fever vaccine in tissue culture". In International Symposium on Yellow Fever and Dengue - Fifty years from the introduction of the 17D strain in Brazil Annals) introduced the use of CEF cells culture in the production of YF 17D vaccine virus. The incentive to use cells culture instead of chicken embryo was to overcome several important problems related to the high egg protein content of the vaccine (up to 250 µg of chicken protein per human dose) and difficulties related to expanding-scale production.

While Lopes, O.S. has cited the use of CEF cells as a potential solution to the above mentioned problems, a very important production feature remained to be solved, namely the low yield of virus produced relative to the amount of seed virus used. This is one reason why yellow fever vaccines are still produced by the viral inoculation of chick embryos in-ovo.

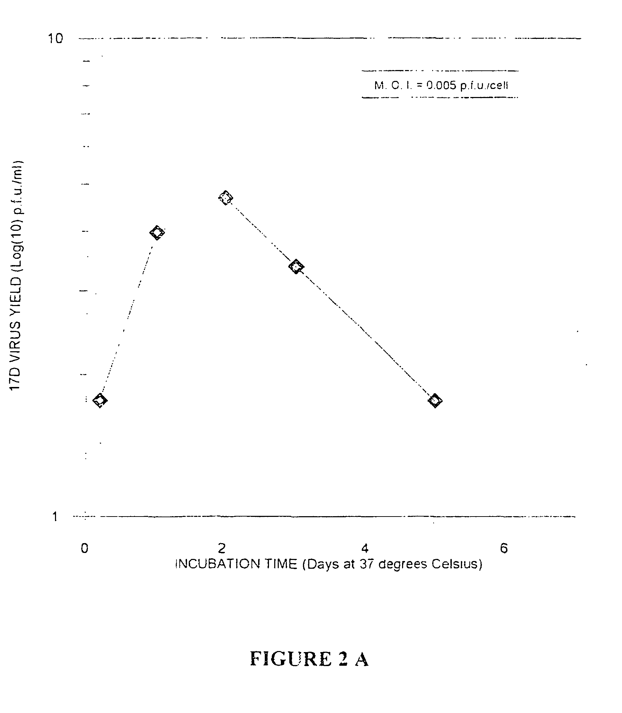

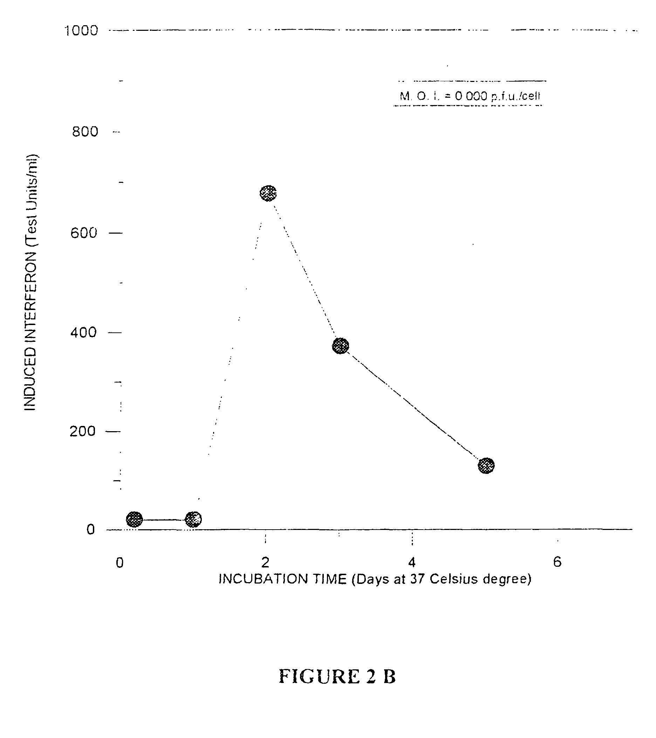

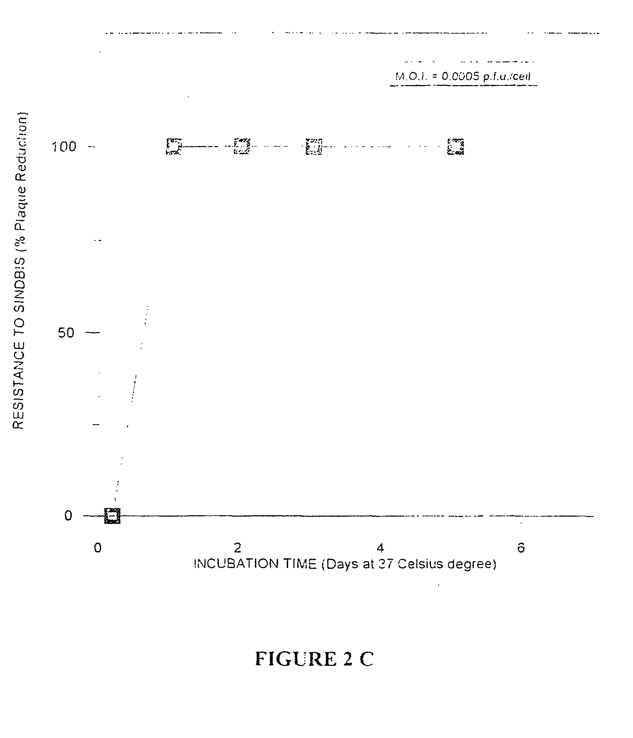

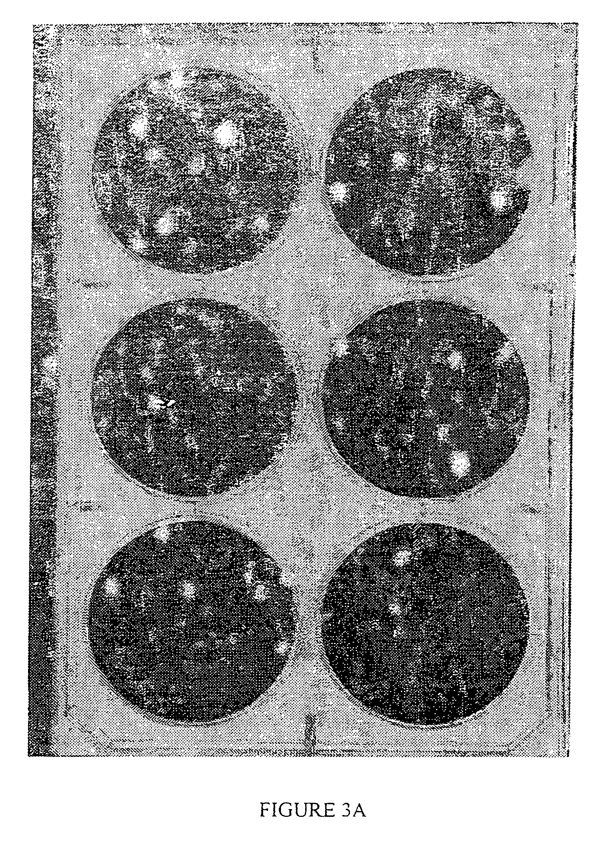

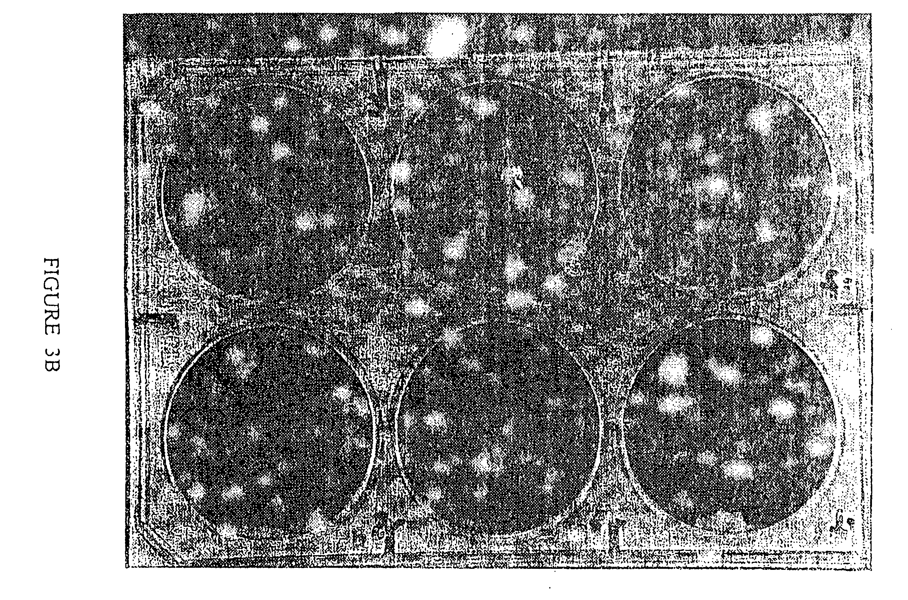

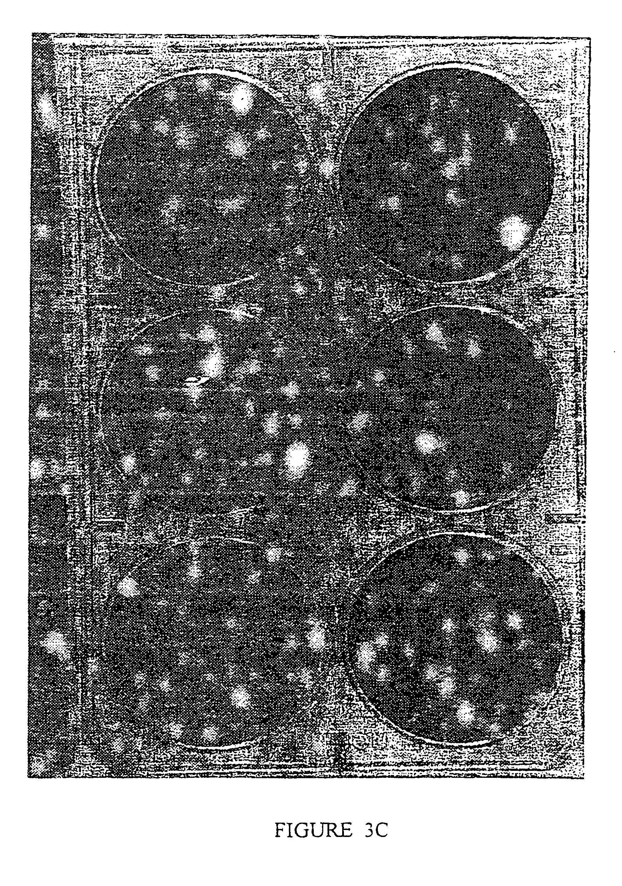

Studies on the replication of the YF 17D virus in CEF were carried out to know which factors cause the reduction in virus yield. The factors investigated were the presence of defective interfering (DI) particles, the induction of interferon and sensitivity to its action and the effect of cell seeding rates. Tables I and II and figures 2 and 3 show the results of experiments using YF 17D-213 virus in CEF This YF 17D substrain was used because it is the virus seed from WHO known to be free of avian leukosys virus complex and its attenuated phenotype and immunogenicity are well characterized in both nonhuman primates and humans. In addition it was the substrain recommended by WHO to be employed in order to establish production of YF 17D vaccine in cultured cells and accordingly it was the virus used by O S. Lopes for the initial studies on the production of YF vaccine in CEF cells which yielded virus attenuated for nonhuman primates in three consecutive production batches. Its passage history is depicted in Figure 1. Vero cells were used as controls (Table ID) since these cells are known to give multiple cycles of replication, complete cytopathology and to be defective in interferon production.

**TABLE I.**

| REPLICATION OF 17D VIRUS IN CONFLUENT CEF AND VERO CELL CULTURES | | | | | | | |
|---|---|---|---|---|---|---|---|
| SEED VIRUS DILITION LOG₁₀ | POST INOCULATION INCUBATION TIME | | | | | | pfu/cell Day 2 Arith. |
| | 5 HOURS | 1 DAY | 2 DAYS | 3 DAYS | 5 DAYS | 7 DAYS | |
| A. VIRUS YIELD FROM CEF (Log₁₀ pfu/ml) | | | | | | | |
| - 1.0 | 2.36 | 5.11 | 5.27 | 4.32 | < 1.76 | < 1.76 | 0.093 |
| - 2.0 | < 1.76 | 3.96 | 4.67 | 3.36 | < 1.76 | <.1.76 | 0.023 |
| - 3.0 | < 1.76 | 3.40 | 4.46 | (2.36) | < 1.76 | < 1.76 | 0.014 |
| - 4.0 | < 1.76 | 2.06 | 4.00 | (2.61) | < 1.76 | < 1.76 | 0.005 |
| - 5.0 | < 1.76 | 1.76 | 2.96 | (2.46) | < 1.76 | < 1.76 | < 0 |
| | n = | 5 | 5 | | | | |
| LINEAR | r = | - 0.985 | - 0.968 | | | | |
| REGRESSION | | | | | | | |
| ANALYSIS | p = | < 0.01 | < 0.01 | | | | |
| | k = | - 0.860 | - 0.529 | | | | |

| B. INTERFERON YIELD FROM CEF (Log₁₀ Units/ml) | | | | | | | |
|---|---|---|---|---|---|---|---|
| - 1.0 | < 1.30 | 1.60 | 3.17 | 3.15 | 2.64 | 2.88 | |
| - 2.0 | < 1.30 | < 1.30 | 2.83 | 2.57 | 2.11 | 2.18 | |
| - 3.0 | < 1.30 | < 1.30 | 2.18 | 1.78 | 1.70 | 1.48 | |
| - 4.0 | < 1.30 | < 1.30 | < 1.30 | 1.48 | 1.48 | 1.48 | |
| - 5.0 | < 1.30 | < 1.30 | < 1.30 | (1.48) | 1.30 | < 1.30 | |
| | n = | | | 4 | | | |
| LINEAR | r = | | | - 0.987 | | | |
| REGRESSION | | | | | | | |
| ANALYSIS | p = | | | < 0.02 | | | |
| | k = | | | - 0.580 | | | |

| C. RESISTANCE OF CEF TO SINDBIS VIRUS (% PLAQUE REDUCTION) | | | | | | | |
|---|---|---|---|---|---|---|---|
| - 1.0 | 0 | 100 | 100 | 100 | 100 | 100 | |
| - 2.0 | 0 | 100 | 100 | 100 | 100 | 100 | |
| - 3.0 | 0 | 30° | 100 | 100 | 100 | 100 | |
| - 4.0 | 0 | | 100 | 100 | 100 | 100 | |
| - 5.0 | 0 | 0 | 13° | 100 | 100 | 100 | |

| D VIRUS YIELD FROM VERO (Log₁₀ Units/ml) | | | | | | | |
|---|---|---|---|---|---|---|---|
| - 1.0 | 2.51 | 3.77 | 5.80 | 7.28 | 6.17 | < 1.76 | 19.05 |
| - 2.0 | < 1.76 | 3.01 | 4.74 | 7.19 | 6.40 | < 1.76 | 15.49 |
| - 3.0 | < 1.76 | (1.76) | 4.11 | 6.66 | 6.59 | < 1.76 | 4.57 |
| - 4.0 | < 1.76 | (1.76) | 3.10 | 5.01 | 7.30 | < 1.76 | 19.95 |
| - 5.0 | < 1.76 | < 1.76 | 2.06 | 4.26 | 7.64 | 4.71 | 43.65 |
| | n = | | 5 | 5 | 5 | | |
| LINEAR | r = | | - 0.997 | - 0.951 | - 0.974 | | |
| REGRESSION | | | | | | | |
| ANALYSIS | p = | | < 0.001 | < 0.1 | < 0.05 | | |
| | k = | | - 0.912 | - 0.822 | - 0.384 | | |
| OBSERVATIONS: (1) PLAQUE SIZE REDUCED | | | | | | | |
| (2) CELL CONCENTRATIONS: CEF → Initial 2 x 10⁶/ml. Final 1 x 10⁶/ml in controls | | | | | | | |
| (3) MOI (MULTIPLICITY OF INFECTION) VERO → Initial 1 x 10⁶/ml. Final 2.2 x 10⁶/ml in controls | | | | | | | |
| CEF → 0.025 pfu/cell at 10^{-1.0} | | | | | | | |

As shown in Table 1A and Figure 2A, CEF gave peak 17D virus yields after 2 days incubation and log₁₀ yields were linearly related to the logarithm of the virus input (r = 0968. p .0.01). Each tenfold decrease in virus input resulted in a 0 53 log decrease in virus yield. No cytopathic effects were observed throughout the 7 days incubation period.

As expected. Vero cells gave similar yields at all virus inputs with the time to maximum titre being inversely related to the log₁₀ concentration of the virus inoculum (Table 1D). On day 3 for example, yield is directly related to virus input (r = + 0.951. p < 0.1) while by day 5 this relationship is inverted (r = - 0.974, p < 0.05). Virus yield from Vero cells were about 200 times greater than those from CEF even at the lowest virus input. A near total cytopathic effect was observed in all infected Vero cell cultures

Virus yields in both cell types were not significantly reduced even at the highest virus input. In multiple virus assays in Vero cells, using high virus concentrations, cytopathic effect was always observed thus no cell "'sparing" occurred. Reduced yield and cell "sparing" at high input represent the basic indicators of defective interfering particles (DI's) These results therefore constitute definitive proof that DI's play no significant role in the replication of the 17D virus.

However, as mentioned above, the interferon system is one of the main mechanisms which limit replication of the 17D YF virus since it is both a good inducer of IF and is sensitive to its action.

In the same experiment as discussed above, interferon (Table IB. Figure 28) was induced at some level in all infected cultures of CEF with yields directly related to virus input (r = 0.987, p < 0.02). Challenging 17D YF infected cultures of CEF with Sindbis (a known IF sensitive virus) at various times post infection (Table IC. Figures 2C and 3) shows that all cultures were totally resistant to challenge by 3 days and to be resistant on day 2 at all except the lowest virus input tested. It was concluded therefore that the induction of interferon and sensitivity to its action are the principal factors influencing the yield of 17D virus in CEF.

All of the YF 17D substrains tested (DD, 5.2/VL and 5.2/DD) were shown to behave in the same manner as 17D-213 virus (discussed above) with respect to interferon induction and sensitivity.

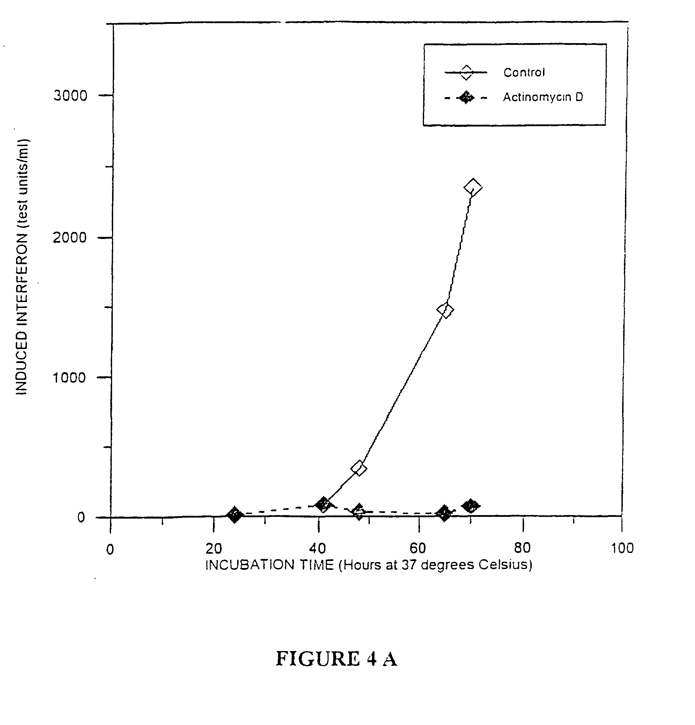

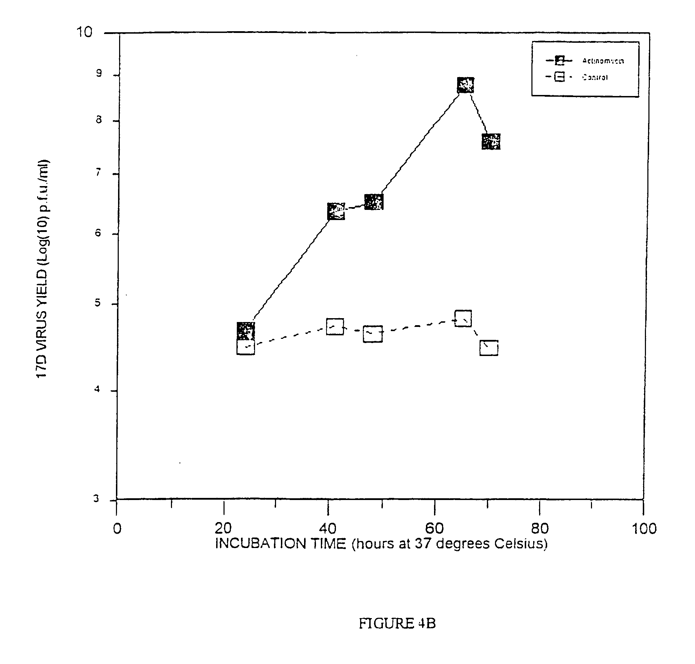

These results demonstrate the necessity of infecting the cells with the virus under specific process conditions to evade the interferon system. Consistently, the use of interferon inhibitory substances would be a possible solution to increase the virus yield. However, said substances are only poorly interferon inhibitory or can be toxic to the cells and are not acceptable as contaminants of vaccines. In this regard, recent studies have also shown that the actinomycin D. a known irreversible inhibitor of DNA dependent RNA synthesis, blocks the interferon response, permiting multiple cycles of replication and increased YF virus yields. Figure 4 shows the influence of the pretreatment of the cells with actinomycin D and then infected with 170 YF virus. This procedure gave a substantial increase in virus yield relative to control cultures. This result, attributable to the inhibition of interferon production, confirms that the interferon system is responsible for low yields. But actinomycin D is relatively toxic to the cells and not acceptable as a contaminant in vaccines.

Thus, the last aspect investigated to overcome the interferon system induction was the cellular density at the time of viral infection.

The results of a typical experiment to test the effect of cell seeding density on the yield of 17DD YF virus from CEF are presented in Table II.

**TABLE II:**

| INFLUENCE OF CELL SEEDING DENSITY ON THE YIELD OF 17D YF VIRUS FROM CEF | | | | |
|---|---|---|---|---|
| Cells/cm² x 10⁴ | Cells/ml x 10⁵ | Log₁₀ pfu/ml | Yield pfu/cell | |
| | | | Log₁₀ | Arith. |
| 1 | 0.25 | (7.11) | 2.71 | 513 |
| 3 | 0.75 | 7.28 | 2.40 | 251 |
| 5 | 1.25 | 6.78 | 1.68 | 47.9 |
| 7 | 1.75 | 6.30 | 1.06 | 11.5 |
| 9 | 2.25 | 6.15 | 0.80 | 6.3 |
| 20 | 5.00 | 5.48 | - 0.22 | 0.6 |
| LINEAR REGRESSION | n = | 5 (values in parenthesis excluded) | | |
| ANALYSIS | r = | 0.993 (p < 0.001) | | |
| (log₁₀ cells/cm² versus log₁₀ pfu/ml) | k = | 2.197 (log₁₀ yield increase for a 1 log reduction in cells) | | |

These results unequivocally demonstrate that the log₁₀ virus yield is inversely and linearly related to the log₁₀ concentration (r = 0.994. p < 0.001) of cells seeded in the range of 3 x 10⁴ to 2 x 10⁵ cells/cm². In this range, a 2.2 log₁₀ increase in virus yield/ml is obtained for each log₁₀ decrease in the cell concentration. At 3 x 10⁴ cells/cm², the density at which the maximal yield per millilitre was obtained, the yield per cell was 251 pfu/cell or 418 fold more than that obtained at 2 x 10⁵ cells/cm². These results definitively prove that the infectious virus yield/ml and yield/cell is inversely related to the cell seeding density Thus, the interferon system can be evaded and efficient production established.

At high cell seeding densities no cytopathology was observed while at low densities, total cell death was observed about one day after peak virus yields. At intermediate densities, partial cytopathology was observed.

According to the invention, one of the major goals in the vaccine virus production was to carry out the cell seeding with the virus at low densities. i.e.. at cell seeding densities lower than 2 x 10⁵ cells/cm², preferentially at densities in the range of 1 x 10⁴- 2 x 10⁵ cells/cm² and more preferentially 1 x 10⁴ - 1 x 10⁵ cells/cm².

Another relevant aspect in vaccine virus production is related to materials used in the culture medium since foreign proteins are not acceptable as components of human vaccines. For example, fetal bovine serum proteins are not acceptable in parenteral products in view of their immunogenicity On the other hand, tests have shown that low virus yields from cell cutures, e.g. CEF, are obtained in the absence of fetal bovine serum (FBS). In experimental studies, yields of 17D YF virus were reduced in about 100 fold in the absence of serum.

Table III shows the replication of YF (17DD substrain) virus and induced resistance to Sindbis virus challenge in the presence and absence of serum.

**TABLE III:**

| REPLICATION OF YF 17D VIRUS AND INDUCED RESISTANCE TO SINDBIS VIRUS CHALLENGE IN THE PRESENCE AND ABSENCE OF SERUM | | |
|---|---|---|
| Seed Dilution | % Resistance to Sindbis at 48 hours | |
| Log₁₀ | With FBS | Without FBS |
| - 1 | 100 | 100 |
| - 2 | 100 | 100 |
| - 3 | 90 | 90 |
| - 4 | 0 | 0 |
| - 5 | 0 | 0 |
| Controls | 0 | 0 |

The results clearly demonstrate that the virus replicates well in the absence of serum and that no significant change occurs in interferon induction. Indeed, the low VF virus yields from CEF in the absence of FBS can be attributed to the virus stabilising effect of the albumen fraction of FBS.

Therefore, according to the invention, medium supplements acceptable as components in parenteral products are used in cell cultures to stabilize the virus produced in order to obtain high virus yields. e.g. human serum albumen (HSA). Other substances acceptable as components in parenteral products, such as peptides, amino acids, proteins, can be used.

According to the invention, the cell culture to be infected by the virus may be any cell insofar as virus can replicate and which is acceptable as a substrate for vaccine production, in particular any cell type which produces interferon in which flaviviruses or recombinants might grow and the same procedure of reducing cell density may be applied. Specific examples include avian embryo cells and mammalian cells such as chicken embryo fibroblasts (CEF), chicken embryo cells (CE), human diploid fibroblasts (eg. MRC-5), monkey kidney cells, fetal Rhesus lung (FRhL) cells etc.

The invention is particularly suitable for the production of Flavivirus and the production of recombinant Flavivirus.

According to the invention, recombinant YF virus as defined in the co-pending Patent Application BR P1 9701774 may be produced, the biomass consisting of avian embryo fibroblast certified cells, in particular SPF (Specific Pathogen Free) chicken embryo fibroblast cells. The co-pending Patent Application BR PI 9701774 is herein mentioned by reference.

The process for the production of YF 17D vaccine described here was established using several substrains of 17D virus. It is, therefore, a procedure for the propagation of 17D viruses in general, be the 17D virus derived from further passaging of any existing 17D substrains or recombinant viruses derived from cloned complementary cDNA through infectious clone technology.

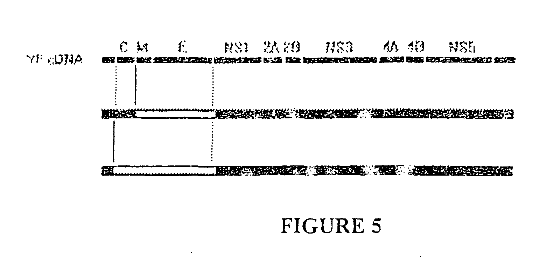

Other recombinant YF 17D viruses may include:
1) chimeric viruses for use in a vaccine preparation after propagation using the methodology of the present invention having a genome comprising nucleic acid sequences encoding at least one structural protein from one flavivirus (represented by the clear areas: see Figure 5) and nucleic acid sequences encoding the remainder of the genome of YF 17D virus to make it functional (represented by the black areas: see Figure 5). The source of YF 17D sequences may be plasmids pYF5'3'IV/G1/2 and pYFM5.2/T3/27 or any other plasmids containing YF 17D virus genomic sequences.
   The schematics of figure 5 shows on the top part the prototype structure of the flavivirus genome. From the 5' terminus the order of the encoded proteins is the following: C-prM/M: E, NS1, NS2A, NS2B, NS3, NS4A, NS4B, NS5. The first 3 proteins constitute the structural proteins, that is, form the virus together with the packaged RNA molecule and were named capsid (C. 12-14kDa), membrane (M. and its precursor prM. 18-22 kDa) and envelope (E.52-54 kDa) all being encoded in the first quarter of the genome. The remainder of the genome codes for the nonstructural proteins (NS) numbered in the order of synthesis from 1 through 5. Three large nonstructural proteins have highly conserved sequences among flaviruses. NS1 (38-41 kDa), NS3 (68-70 kDa) and NS5 (100-103 kDa). No role has yet been assigned to NS1 but NS3 has been shown to be bifunctional with a protease activity needed for the processing of the polyprotein at sites the cellular proteases will not act and a nucleotide triphosphatase/helicase activity being therefore also associated with viral RNA replication. NS5, the largest and most conserved protein, contains several sequence motifs believed to be common to viral RNA polymerases. The 4 small proteins NS2A, NS2B, NS4A and NS4B are poorly conserved in their amino acid sequences but not in their pattern of multiple hydrophobic stretches. NS2A has been shown to be required for proper processing of NS1 whereas NS2B has been shown to associate with the protease acivity of NS3 to process itself from NS3 and produce NS4B. Since viral RNA synthesis takes place in the cytosol in association with RER (Rough Endoplasmic Reticulum) membranes it has been postulated that these hydrophobic proteins would be embedded in membranes and through protein-protein interactions participate in viral replication complexes together with NS3 and NS5. The short 5' (118 nucleotides) and 3' (511 nucleotides) untranslated regions contain nucleotide sequence elements some of which are conserved among the flaviviruses and believed to be involved in plus and minus strand RNA replication and plus strand RNA packaging. Chimeric flaviviruses may include the capsid C through the envelope E protein, prM-E or E alone of one flavivirus and the remainder derived from a second flavivirus as indicated in the lower two parts of figure 5. Fusion between the C and prM proteins of any two flaviviruses, including YF, can be made at the carboxi terminus of C (its last 20 amino acids), at the cleavage site of the viral protease or at the signalase cleavage site. Adjacent to the first nucleotide of the viral genome, as a cloned cDNA bacterial plasmid, is a bacterial phage polymerase promoter sequence (SP6, T7 or T3) which allows the efficient *in vitro* synthesis of infectious capped RNA transcripts. These, in their turn, originate virus after RNA transfection of cultured cells.
2) YF17D viruses expressing defined epitopes, protein domains or entire proteins of other pathogens related to specific aspects of humoral or cellular immune response which can be used as a vaccine against said disease inserted within the structural or nonstructural protein genes or the noncoding regions of YF 17D virus genome.
3) YF17D viruses expressing from a sub-genomic RNA defined epitopes, protein domains or entire proteins of other pathogens related to specific aspects of humoral or cellular immune response which can be used as a vaccine against said disease.

In all cases above (1-3) the recombinant virus still contains most if not all of the YF 17D genome and therefore all major steps towards viral replication in the host cell will be the same as the original 17D virus. This includes cytoplasmic replication, the participation of YF 17D viral proteins in viral RNA synthesis (plus and minus strand), polyprotein processing, capsid formation with RNA packaging, virus assembly and budding. Since all major aspects which lead to a productive infection are kept regardless of the extent of genetic modification in the cases specified above, the methodology described here will apply.

Therefore, according to one of the embodiments of the present invention, YF vaccine virus is produced under Good Manufacturing Practices (GMP) by infecting cells certified for human vaccine production and recovering the virus.

In a preferred embodiment of the present invention, to accomplish the virus propagation in cells certified for the production of human vaccines, primary cultures of chicken embryo fibroblasts (CEF) are used in all production steps. Cells are available and prepared according to WHO (World Health Organization) Guidelines as described by several S.O.Ps. (Standard Operational Procedures). Particularly in the case of YF virus, the production of YF 17D vaccine in CEF cultures, although at low yields, gave 3 lots of experimental vaccine which passed all tests including neurovirulence for monkeys.

The virus is prepared in primary cultures of chicken embryo fibroblasts (CEF) using eggs derived from SPF (Specific Pathogen Free) flocks. The cultures of chick embryo fibroblasts (CEF) cells can be made in different forms. e.g., monolayers: in suspension, in a suitable bioreactor; adsorbed to microcarriers. The preparation of CEF cells is known by those skilled in the art. Cell cultures are set up in a suitable medium and are later infected. Viruses are recovered after incubation by centrifugation or filtration to remove cellular debris. To the supernatant containing the virus a stabilizer was added which is, for those skilled in the art, known to enhance the stability of viral infectivity during freezing, thawing and subsequent manipulations. Viruses are stored at - 45°C or below.

The following examples are illustrative of the invention and represent preferred embodiments. Those skilled in the art may know, or be able to find using no more than routine experimentation, to employ other appropriate materials and techniques, such as the above mentioned cells and virus production methods.

### EXAMPLE 1

The purpose of this example is to obtain Flavivirus, in particular 17D Yellow Fever Virus, in cultures of chick embryo fibroblasts (CEF) cells in monolayers as follows:
(1) SPF chick embryo tissue is converted to a cell suspension by enzymatic treatment (Day 0). This preparation is known by those skilled in the art;
(2) the cells are recovered and suspended in a suitable medium followed bv seeding into monolayer culture vessels (flasks, roller bottles, multisurface propagator/reactor, etc.) at 0.1 to 1.2 ml/cm² to give to to 10^{5.3} cells/cm²;
(3) the culture is incubated for 12 to 72 hours at 30 to 40 °C;
(4) the medium is removed from the monolayers and virus is added in a small volume of a suitable medium at the rate of 0.2 - 0.0001 infectious units per cell (CCID₅₀, LD₅₀, pfu);
(5) the virus is adsorbed for 0.5 to 4 hours at 20 to 40°C (Day 1);
(6) a suitable maintenance medium is added at 0.1 to 1.0 ml/cm² with or without removal of the seed preparation and re-incubation at 30 - 40°C for 15 to 30 hours;
(7) the maintenance medium is removed, and the monolayers washed once or more times and replaced with serum free maintenance medium;
(8) the monolayers are re-incubated at 30 - 40°C (Day 2); after an appropriate incubation time (e.g. 36 to 72 hours post inoculation at 37° C), the maintenance medium containing virus is harvested (Day 3); and
(9) the virus is stored at -45 to -196°C.

Multiple harvests of virus containing medium may be carried out by total or partial removing of the medium, at any desired interval.

### EXAMPLE 2

The purpose of this example is to demonstrate the virus stabilizing effect of albumen.

A process as for example 1 in which human serum albumin is incorporated into the maintenance medium at 0.01 to 10 µg/ml at steps 6. 7 and 9. The Tables IV and V show the results.

**TABLE IV:**

| STABILITY OF YELLOW FEVER VIRUS IN MEDIUM CONTAINING VARIED CONCENTRATIONS OF HUMAN SERUM ALBUMEN | | | | | | |
|---|---|---|---|---|---|---|
| Albumen concentration | | Log₁₀ pfu/ml | | | Half Life | |
| % w/v | | Hours at 37°C | | | Loss/hr | Hours |
| | 0.25 | 1.08 | 2.37 | 3.50 | | |
| 0.500 | TNC* | TNC* | 2.51 | 2.31 | 0.18 | 1.7 |
| 0.250 | TNC* | TNC* | 2.51 | 2.40 | 0.10 | 3.0 |
| 0.125 | TNC* | TNC* | 2.55 | 2.33 | 0.19 | 1.6 |
| 0.063 | TNC* | TNC* | 2.48 | 2.33 | 0.13 | 2.3 |
| 0.031 | TNC* | TNC* | 2.50 | 2.00 | 0.27 | 1.11 |
| 0.000 | TNC* | 1.00 | <0.40 | <0.40 | <1.80 | <0.40 |

| | | | | | | |
|---|---|---|---|---|---|---|
| * Too Numbered to Count | | | | | | |

These results are clearly approximations but there is a strong indication that 0.03125% w/v HSA is insufficient to protect virus infectivity. The mean rate of loss in the presence of 0.0625 to 0.2500% w/v albumen is 0. 15 log₁₀/hour equivalent to a half life of 2 hours.

**TABLE V:**

| INFLUENCE OF HUMAN SERUM ALBUMEN (HSA) AND HARVEST TIME ON THE YIELD OF YF 17D VIRUS FROM CEF | | | |
|---|---|---|---|
| HSA % w/v | Hours Post Inoculation | Log₁₀ pfu/ml | Arithmetic pfu/ml |
| 0.06 | 29 | 4.83 | 0.6 |
| | 44 | 6.71 | 85.1 |
| | 48 | 6.82 | 109.6 |
| | 53 | 6.69 | 81.3 |
| | 68 | 6.04 | 18.2 |
| | | | |
| 0.20 | 29 | 5.00 | 1.7 |
| | 44 | 6.70 | 83.2 |
| | 48 | 6.95 | 147.9 |
| | 53 | 6.85 | 117.5 |
| | 68 | 6.23 | 28.2 |

As shown in Table V, peak titres were observed at 48 hours in the presence of 0.06 and 0.2% w/v. Yield per cell is highly satisfactory with values in the region of 100 pfu/cell. Clearly therefore, HSA, which is a wholly acceptable vaccine component, gives excellent virus yields. These values should be objectively high since the samples are diluted ½ (0.3 log₁₀) in stabilizer and assayed in a system which is 0.2 to 0.3 log₁₀ less sensitive than that used to control virus which is the final product produced by the current process; i.e. by using the whole chicken embryo. On this basis, correction of the titres shown in Tables IV and V gives values in of 7.0 about log₁₀ pfu/ml and are equal to those obtained in the production of the whole chick embryo vaccine.

### Example 3

The purpose of this example is to illustrate the production of Flavivirus, in particular 17D Yellow Fever Virus, produced under Good Manufacturing Practices (GMP) by infecting cells certified for human vaccine preparation.

A total of 21 roller bottles with a surface area of 680 cm² each was used for each batch. The WHO (World Health Organization) Guidelines require that 10% of flasks or a minimal volume of 500 ml of culture be used as control. For this purpose 7 flasks of 175 cm² surface area each with stationary cultures were used for each batch.

The preparation of each lot was accomplished by following the same pocedure as described in Example 1.

Primary CEF cells obtained from eggs derived from SPF (Specific Pathogen Free) flocks were used in all lots. Seed virus 17DD-LOT 102/84 was used to inoculate all cell culture.

Virus were recovered by pooling the medium present in every three roller into centrifuge tube and spinning at low speed to remove cellular debris. The supernatant was aspirated into flasks containing stabilizer at 1.1 ratio and frozen slowly by rotating on an ethanol dry ice bath after removal of all quality control aliquots. All viruses are stored as indicated in Example 1.

The production data are presented in the Table VI below.

**TABLE VI:**

| PRODUCTION AND QUALITY CONTROL DATA OF YELLOW FEVER VACCINE PRODUCED IN CHICKEN EMBRYO FIBROBLASTS (CEF) UNDER GMP | | |
|---|---|---|
| **LOT** | **Production Date** | **Titer (log**_{**10**} **pfu/ml)** |
| YFCEF-LOT 01 | 02/02/98 | 6.79 |
| YFCEF-LOT 02 | 03/02/98 | 6.68 |
| YFCEF-LOT 03 | 04/02/98 | 6.66 |
| YFCEF-LOT 04 | 05/02/98 | 6.75 |

Each lot was tested for sterility, potency and adventitious agents with satisfactory results.

### EXAMPLE 4

The purpose of this example is to describe the preparation of recombinant YF virus including chimeric viruses by using the process of the present invention.

The YF 17D virus of the Patent Application BR PI 9701774 is regenerated from plasmids pYF5'3'IV/G1/2 and pYFM5.2/T3/27 and will be hereinafter refered to as YFiv5.2/DD. Deposit of plasmids pYF5'3'IV/G1/2 and pYFM5.2/T3/27 has been made at the American Type Culture Collection and they are identified by ATCC Accession No. 97771 and 97772, respectively.

### a) RNA transfection:

The transfection of CEF cells is carried out with Lipofect AMINE® (Life Technologies catalogue # 18324-012). It is a 3:1 (w/w) liposome formulation of the polycationic lipid 2.3-dioleyloxy-N-[2(sperminecarboxiamido)ethyl]-N-N, dimethyl-2,3-bis(9-octadecenyloxy)-1-ropanaminium trifluoro acetate) and the neutral dioleoylphosphatidyl ethanolamine (DOPE) in membrane-filtered water) at a concentration of 20 µg/ml in RNase-free PBS. PBS - phosphate buffer saline- is prepared and sterilized bv autoclaving at 121°C for 20 minutes. The LipofectAMINE® is pipeted into a polystyrene 5 ml tube containing PBS. Primary CEF cells are seeded and used 24 hours post seeding.

The transcription mix is added to the cell culture monolayer dropwise. Cells are incubated for 20 minutes at room temperature. Thereafter, the mix is removed by aspiration, washed with PBS and incubated for 72 hours in 199 medium.

The culture supernatant constitute the viral stock after addition of stabilizer. The viral stock is tested for sterility, toxicity, potency and for the presence of adventitious agents. The viral stock is the original seed lot.

The specific infectivity of the transcripts is deduced from experiments in which serial dilutions of the RNA are tranfected into Vero cells and these are overlaid with semisolid medium. Staining with crystal violet for the counting of plaques and knowing the amount of RNA. as determined by measuring the OD (optical density) at 260 nm, will allow the determination of specific infectivity of the transcripts (PFU/µg total RNA).

This determination is relevant to establishing the multiplicity of infection events that lead to the original stock. It ensures an acceptable number of events equivalent to infection with live virus in order to reduce the probabitily of accumulation of mutations in the viral genome given the high number ot replication cycles due to low RNA input.

### b) Preparation of seed lots

All lots were prepared in primary cultures of CEF cells using eggs derived from SPF (Specific Pathogen Free) flocks. Viruses are recovered by pooling the supernatant present in each flask into centrifuge bottles and spinning at low speed to remove cellular debris. The supernatant is aspirated into flasks containing stabilizer at 1:1 ratio and frozen slowly by rotating on an ethanol dry ice bath after removal of all quality control aliquots. All viruses are stored as indicated above.

### b.1) Preparation of the original seed lot

The original seed lot consists of the material derived from 3 separate transcription/transfection experiments carried out on different days with different batches of primary CEF cultures.

Primary chicken embryo fibroblasts were seeded. A total of disposable T-flasks of 175 cm², containing *in vitro* transcribed RNA were transfected into CEF cells using LipofectAmine®. Each T-flask provided a total of 80 ml of culture supernatant. In three separate transfections performed at different days and therefore with different lots of CEF cells titers for the original seed lots were: T1. 10^{4.66} (4.66 log₁₀ pfu/ml); T2, 10^{4.87} (4.87 log₁₀ pfu/ml); T3. 10^{5.46} (5.46 log₁₀ pfu/ml). Each lot provided a total volume of 480 ml of original virus. Eighty ml were used for quality control remaining 400 ml for the preparation of primary seed lot(s).

### b.2) Preparation of primary seed lot

Two primary seed lots were prepared and named LP1 and LP2. LP1 derives from original seed lot T3 whereas LP2 derives from T2. Each was tested for sterility, potency and adventitious agents with satisfactory results.

The obtained volumes and titers are:

| Seed lot | Volume (ml) | Titer (log₁₀ pfu/ml) |
|---|---|---|
| LP1 | 1.200 | 6.22 |
| LP2 | 1.600 | 6.20 |
| pfu= plaque-forming unit | | |

### b.3) Secondary seed lots

Three secondary seed lots were prepared and named LS1, LS2 and LS3. LS1 and LS2 derive from primary seed lot LP1 whereas LS3 derives from LP2. Each was tested for sterility, potency and adventitious agents with satisfactory results.

The obtained volumes and titers are:

| Seed lot | Volume (ml) | Titer (log₁₀ pfu/ml) |
|---|---|---|
| LS1 | 5.200 | 6.20 |
| LS2 | 5.600 | 6.05 |
| LS3 | 5.200 | 6.73 |

Each of these seed lots should suffice for YF vaccine production using current manufacturing methodology (embryonated eggs) or the cellular system (CEF cells) for nearly 50 years at a rate of at least 50 million doses/vear.

## Claims

1. Process for the production of a Flavivirus in a cell culture, the process comprising:
(a) preparing a culture of cells which are permissive to the virus and acceptable as a substrate for vaccine production;
(b) suspending the cells in a suitable medium followed by cell seeding at a density lower than 2x10⁵ cells/cm²;
(c) incubating the cell culture at 30 to 40°C for a period of time between 12 and 72 hours;
(d) removing the medium from the cell culture of step (c) and inoculating the cells with seed virus;
(e) incubating the cell culture of step (d) at 20 to 40°C for a period of time between 12 and 144 hours;
(f) removing the medium, washing the cells one or more times and replacing the medium;
(g) incubating the cell culture of step (f) at 30 to 40°C for a period of time between 12 and 144 hours;
(h) at least partially harvesting the culture supernatant containing virus; and
(i) storing the virus at a temperature of -45°C or lower.

2. Process for the production of a recombinant yellow fever (YF) 17D virus in a cell culture, the process comprising:
(a) preparing a culture of cells which are permissive to the virus and acceptable as a substrate for vaccine production;
(b) cell seeding at a density lower than 2x10⁵ cells/cm²;
(c) transfecting the cells with nucleic acids suitable for the production of a recombinant YF 17D virus in a cell culture, by treatment with synthetic lipids or electroporation;
(d) incubating the cell culture at 30 to 40°C for a period of time between 12 and 72 hours;
(e) removing the medium from the cell culture of step (d) and inoculating the cells with seed virus;
(f) incubating the cell culture of step (e) at 20 to 40°C for a period of time between 12 and 144 hours;
(g) removing the medium, washing the cells one or more times and replacing the medium;
(h) incubating the cell culture of step (g) at 30 to 40°C for a period of time between 12 and 144 hours;
(i) at least partially harvesting the culture supernatant containing virus; and
(j) storing the virus at a temperature of -45°C or lower.

3. Process according to claim 1 or 2 wherein the culture is incubated at steps (e) and (g) of claim 1 or steps (f) and (h) of claim 2 from between 12 to 72 hours.

4. Process according to any one of the preceding claims wherein multiple harvests of virus containing medium are carried out, at any desired interval, by replacing the removed medium and re-incubating the culture for a period of time between 12 and 144 hours.

5. Process according to claim 4 wherein the culture is re-incubated for a period of time between 12 and 72 hours.

6. Process according to any one of the preceding claims wherein any cell debris and whole cells are removed from the harvested virus.

7. Process according to any one of the preceding claims wherein the virus is inactivated.

8. Process according to any one of the preceding claims wherein the cell seeding is carried out at densities in the range of 1x10⁴ - 2x10⁵ cells/cm².

9. Process according to claim 8 wherein the cell seeding is carried out at a density in the range of 1x10⁴ - 1x10⁵ cells/cm².

10. Process according to any one of the preceding claims wherein stabilizer is added to the supernatant at step (h) of claim 1 or step (i) of claim 2.

11. Process according to claim 10 wherein the stabilizer is a substance acceptable as component in parenteral products and is human serum albumen (HSA), a peptide, an amino acid or a protein and/or a mixture thereof.

12. Process according to any of claims 1 and 3 to 11 wherein the Flavivirus is Yellow Fever virus.

13. Process according to any one of claims 2 to 11 wherein the recombinant virus is a chimeric virus having a genome comprising a nucleic acid sequence encoding at least one structural protein from a flavivirus and a nucleic acid sequence encoding the remainder of the genome of a YF 17D virus to make the recombinant virus functional.

14. Process according to any one of the preceding claims wherein the cells are chicken embryo cells or mammalian cells which are interferon-producing cells when submitted to viral infection.

15. Process according to claim 14 wherein the cells are chicken embryo fibroblasts (CEF), chicken embryo cells (CE), human diploid fibroblasts, monkey kidney cells or fetal Rhesus lung (FRhL).

16. Process according to claim 15 wherein the human diploid fibroblasts are MRC-5 cells.

17. Process according to any one of the preceding claims wherein the cells are primary cells or cells which have been further passaged.

18. Process according to any one of the preceding claims wherein the virus is a wild, attenuated or recombinant virus.

19. Process according to claim 18 wherein the virus is Yellow Fever virus.

20. Process according to claim 19 wherein the Yellow Fever virus is attenuated.

21. Process according to claims 19 or 20 wherein the Yellow Fever virus is the YF 17D virus strain and substrains thereof.

## Patentansprüche

1. Verfahren zur Herstellung eines Flavivirus in einer Zellkultur, wobei das Verfahren aufweist:
(a) Bereiten einer Kultur von Zellen, die für das Virus permissiv sind und als ein Substrat zur Impfstoff-Herstellung annehmbar sind;
(b) Suspendieren der Zellen in einem geeigneten Medium, gefolgt von Ansetzen einer Zellkultur mit einer geringeren Dichte als 2x10⁵ Zellen/cm²;
(c) Inkubieren der Zellkultur bei 30-40° C für eine Zeitdauer zwischen 12 und 72 Stunden;
(d) Entfernen des Mediums aus der Zellkultur von Schritt (c) und Inokulieren der Zellen mit Beimpfungsvirus;
(e) Inkubieren der Zellkultur von Schritt (d) bei 20-40° C für eine Zeitdauer zwischen 12 und 144 Stunden;
(f) Entfernen des Mediums, einmaliges oder mehrmaliges Waschen der Zellen und Ersetzen des Mediums;
(g) Inkubieren der Zellkultur von Schritt (f) bei 30-40° C für eine Zeitdauer zwischen 12 und 144 Stunden;
(h) zumindest teilweise Entnehmen des Virus enthaltenden Kultur-Überstands; und
(i) Aufbewahren des Virus bei einer Temperatur von -45° C oder niedriger.

2. Verfahren zur Herstellung eines rekombinanten Gelbfieber (YF)-Virus17D in einer Zellkultur, wobei das Verfahren aufweist:
(a) Bereiten einer Kultur von Zellen, die für das Virus permissiv sind und als ein Substrat zur Impfstoff-Herstellung annehmbar sind;
(b) Ansetzen einer Zellkultur mit einer geringeren Dichte als 2x10⁵ Zellen/cm²;
(c) Transfizieren der Zellen mit Nucleinsäuren, die zur Herstellung eines rekombinanten YF-Virus 17D in einer Zellkultur geeignet sind, durch Behandlung mit synthetischen Lipiden oder Elektroporation;
(d) Inkubieren der Zellkultur bei 30-40° C für eine Zeitdauer zwischen 12 und 72 Stunden;
(e) Entfernen des Mediums aus der Zellkultur von Schritt (d) und Inokulieren der Zellen mit Beimpfungsvirus;
(f) Inkubieren der Zellkultur von Schritt (e) bei 20-40° C für eine Zeitdauer zwischen 12 und 144 Stunden;
(g) Entfernen des Mediums, einmaliges oder mehrmaliges Waschen der Zellen und Ersetzen des Mediums;
(h) Inkubieren der Zellkultur von Schritt (g) bei 30-40° C für eine Zeitdauer zwischen 12 und 144 Stunden;
(i) zumindest teilweise Entnehmen des Virus enthaltenden Kultur-Überstands; und
(j) Aufbewahren des Virus bei einer Temperatur von -45° C oder niedriger.

3. Verfahren nach Anspruch 1 oder 2, bei dem die Kultur in den Schritten (e) und (g) von Anspruch 1 oder den Schritten (f) und (h) von Anspruch 2 zwischen 12 und 72 Stunden inkubiert wird.

4. Verfahren nach irgendeinem der vorangehenden Ansprüche, bei dem Mehrfachentnahmen von Virus enthaltendem Medium in beliebigen gewünschten Intervallen durchgeführt werden, indem das entfernte Medium ersetzt wird und die Kultur erneut für eine Zeitdauer zwischen 12 und 144 Stunden inkubiert wird.

5. Verfahren nach Anspruch 4, bei dem die Kultur für eine Zeitdauer zwischen 12 und 72 Stunden erneut inkubiert wird.

6. Verfahren nach irgendeinem der vorangehenden Ansprüche, bei dem jegliche Zellreste und vollständige Zellen von dem entnommenen Virus entfernt werden.

7. Verfahren nach irgendeinem der vorangehenden Ansprüche, bei dem das Virus inaktiviert wird.

8. Verfahren nach irgendeinem der vorangehenden Ansprüche, bei dem das Ansetzen der Zellkultur bei Dichten in dem Bereich von 1x 10⁴ bis 2x10⁵ Zellen/cm² durchgeführt wird.

9. Verfahren nach Anspruch 8, bei dem das Ansetzen der Zellkultur bei einer Dichte in dem Bereich von 1x10⁴ bis 1x10⁵ Zellen/cm² durchgeführt wird.

10. Verfahren nach irgendeinem der vorangehenden Ansprüche, bei dem in Schritt (h) von Anspruch 1 oder in Schritt (i) von Anspruch 2 dem Überstand Stabilisator zugesetzt wird.

11. Verfahren nach Anspruch 10, bei dem der Stabilisator eine Substanz ist, die als eine Komponente in parenteralen Produkten annehmbar ist, und die Humanserumalbumin (HSA), ein Peptid, eine Aminosäure oder ein Protein und/oder ein Gemisch davon ist.

12. Verfahren nach irgendeinem der Ansprüche 1 und 3 bis 11, bei dem das Flavivirus Gelbfieber-Virus ist.

13. Verfahren nach irgendeinem der Ansprüche 2 bis 11, bei dem das rekombinante Virus ein chimäres Virus mit einem Genom ist, das eine Nucleinsäure-Sequenz, die mindestens ein Strukturprotein von einem Flavivirus kodiert, und eine Nucleinsäure-Sequenz, die den Rest des Genoms eines YF-Virus 17D kodiert, aufweist, um das rekombinante Virus funktionsfähig zu machen.

14. Verfahren nach irgendeinem der vorangehenden Ansprüche, bei dem die Zellen Hühnerembryo-Zellen oder Säugetier-Zellen sind, die Interferon erzeugende Zellen sind, wenn sie einer Virusinfektion ausgesetzt werden.

15. Verfahren nach Anspruch 14, bei dem die Zellen Hühnerembryo-Fibroblasten (CEF), Hühnerembryo-Zellen (CE), menschliche diploide Fibroblasten, Affen-Nierenzellen oder fetale Rhesus-Lungenzellen (FRhL) sind.

16. Verfahren nach Anspruch 15, bei dem die menschlichen diploiden Fibroblasten MRC-5-Zellen sind.

17. Verfahren nach irgendeinem der vorangehenden Ansprüche, bei dem die Zellen primäre Zellen oder Zellen, die weiter subkultiviert wurden, sind.

18. Verfahren nach irgendeinem der vorangehenden Ansprüche, bei dem das Virus eine Virus-Wildform, ein abgeschwächtes oder rekombinantes Virus ist.

19. Verfahren nach Anspruch 18, bei dem das Virus Gelbfieber-Virus ist,

20. Verfahren nach Anspruch 19, bei dem das Gelbfieber-Virus abgeschwächt wird.

21. Verfahren nach Anspruch 19 oder 20, bei dem das Gelbfieber-Virus der YF 17D-Virusstamm und Unterstämme davon ist.

## Revendications

1. Procédé de production d'un flavivirus en culture cellulaire, lequel procédé comporte les étapes suivantes :
a) préparer une culture de cellules qui sont permissives au virus et admissibles en tant que substrat pour la production de vaccins ;
b) mettre les cellules en suspension dans un milieu approprié, puis ensemencer ces cellules à une densité inférieure à 2.10⁵ cellules par cm² ;
c) faire incuber la culture cellulaire à une température de 30 à 40 °C pendant un laps de temps de 12 à 72 heures ;
d) retirer le milieu de la culture cellulaire de l'étape (c) et inoculer aux cellules le virus de semence ;
e) faire incuber la culture cellulaire de l'étape (d) à une température de 20 à 40 °C pendant un laps de temps de 12 à 144 heures ;
f) éliminer le milieu, laver les cellules à une ou plusieurs reprises et remplacer le milieu ;
g) faire incuber la culture cellulaire de l'étape (f) à une température de 30 à 40 °C pendant un laps de temps de 12 à 144 heures ;
h) recueillir au moins une partie du surnageant de culture qui contient le virus ;
i) et conserver le virus à une température inférieure ou égale à -45 °C.

2. Procédé de production en culture cellulaire d'un virus de la fièvre jaune (YF) 17D recombiné, lequel procédé comporte les étapes suivantes :
a) préparer une culture de cellules qui sont permissives au virus et admissibles en tant que substrat pour la production de vaccins ;
b) ensemencer les cellules à une densité inférieure à 2.10⁵ cellules par cm² ;
c) transfecter les cellules avec des acides nucléiques appropriés pour la production en culture cellulaire d'un virus YF 17D recombiné, par traitement avec des lipides synthétiques ou par électroporation ;
d) faire incuber la culture cellulaire à une température de 30 à 40 °C pendant un laps de temps de 12 à 72 heures ;
e) retirer le milieu de la culture cellulaire de l'étape (d) et inoculer aux cellules le virus de semence ;
f) faire incuber la culture cellulaire de l'étape (e) à une température de 20 à 40 °C pendant un laps de temps de 12 à 144 heures ;
g) éliminer le milieu, laver les cellules à une ou plusieurs reprises et remplacer le milieu ;
h) faire incuber la culture cellulaire de l'étape (g) à une température de 30 à 40 °C pendant un laps de temps de 12 à 144 heures ;
i) recueillir au moins une partie du surnageant de culture qui contient le virus ;
j) et conserver le virus à une température inférieure ou égale à -45 °C.

3. Procédé conforme à la revendication 1 ou 2, dans lequel, dans les étapes (e) et (g) du procédé défini dans la revendication 1 ou dans les étapes (f) et (h) du procédé défini dans la revendication 2, on fait incuber la culture pendant un laps de temps de 12 à 72 heures.

4. Procédé conforme à l'une des revendications précédentes, dans lequel on effectue plusieurs récoltes de milieu contenant le virus, à tout intervalle voulu, en remplaçant le milieu éliminé et en reprenant l'incubation de la culture pour un laps de temps de 12 à 144 heures.

5. Procédé conforme à la revendication 4, dans lequel on reprend l'incubation de la culture pour un laps de temps de 12 à 72 heures.

6. Procédé conforme à l'une des revendications précédentes, dans lequel on élimine de la récolte de virus tous les débris cellulaires et cellules entières.

7. Procédé conforme à l'une des revendications précédentes, dans lequel le virus est inactivé.

8. Procédé conforme à l'une des revendications précédentes, dans lequel on ensemence les cellules à une densité de 1.10⁴ à 2.10⁵ cellules par cm².

9. Procédé conforme à la revendication 8, dans lequel on ensemence les cellules à une densité de 1.10⁴ à 1.10⁵ cellules par cm².

10. Procédé conforme à l'une des revendications précédentes, dans lequel, dans l'étape (h) du procédé défini dans la revendication 1 ou dans l'étape (i) du procédé défini dans la revendication 2, on ajoute un stabilisant au surnageant.

11. Procédé conforme à la revendication 10, dans lequel le stabilisant est une substance admissible en tant que composant de produits parentéraux, qui est de l'albumine sérique humaine (HSA), un peptide, un acide aminé ou une protéine, et/ou un mélange de tels composants.

12. Procédé conforme à l'une des revendications 1 et 3 à 11, dans lequel le flavivirus est le virus de la fièvre jaune.

13. Procédé conforme à l'une des revendications 2 à 11, dans lequel le virus recombiné est un virus chimérique dont le génome comporte une séquence d'acide nucléique qui code au moins une protéine de structure d'un flavivirus et une séquence d'acide nucléique qui code le reste du génome du virus YF 17D pour rendre fonctionnel le virus recombiné.

14. Procédé conforme à l'une des revendications précédentes, dans lequel les cellules sont des cellules d'embryon de poulet ou des cellules de mammifère qui deviennent productrices d'interféron lorsqu'elles sont infectées par un virus.

15. Procédé conforme à la revendication 14, dans lequel les cellules sont des fibroblastes d'embryon de poulet (CEF), des cellules d'embryon de poulet (CE), des fibroblastes diploïdes humains, des cellules de rein de singe ou des cellules foetales de poumon de singe Rhésus (FRhL).

16. Procédé conforme à la revendication 15, dans lequel les fibroblastes diploïdes humains sont des cellules MRC-5.

17. Procédé conforme à l'une des revendications précédentes, dans lequel les cellules sont des cellules primaires ou des cellules qui ont subi un passage.

18. Procédé conforme à l'une des revendications précédentes, dans lequel le virus est un virus sauvage, un virus atténué ou un virus recombiné.

19. Procédé conforme à la revendication 18, dans lequel le virus est le virus de la fièvre jaune.

20. Procédé conforme à la revendication 19, dans lequel le virus de la fièvre jaune a été atténué.

21. Procédé conforme à la revendication 19 ou 20, dans lequel le virus de la fièvre jaune est la souche virale YF 17D ou l'une de ses sous-souches.
